Europäisches Patentamt

European Patent Office

Office européen des brevets

(11) Publication number: **0 388 102**
**A2**

(12) **EUROPEAN PATENT APPLICATION**

(21) Application number: **90302574.0**

(22) Date of filing: **09.03.90**

(51) Int. Cl.5: **A61M 5/145**

(30) Priority: **16.03.89 GB 8906112**

(43) Date of publication of application:
**19.09.90 Bulletin 90/38**

(84) Designated Contracting States:
**BE DE FR GB IT NL SE**

(71) Applicant: **The BOC Group plc**
**Chertsey Road**
**Windlesham Surrey GU20 6HJ(GB)**

(72) Inventor: **Earle, Stephen Richard**
**134 Peckover Drive**
**Pudsey, West Yorkshire LS28 8EG(GB)**

(74) Representative: **Gough, Peter et al**
**c/o THE BOC GROUP PLC Patent Department**
**Chertsey Road**
**Windlesham Surrey GU20 6HJ(GB)**

(54) **Improvements in infusion pumps.**

(57) An infusion pump 1 includes a body case 2 and a clamp 4 mounted for pivotal movement on the body case 2 between a closed clamping position and an open position. A spring 8 biases the clamp 4 towards its closed clamping position. A potentiometer 22 senses angular movement of the clamp 4 and hence the size of a syringe 100 mounted in the infusion pump 1.

FIG.1.

EP 0 388 102 A2

## IMPROVEMENTS IN INFUSION PUMPS

The present invention relates to fluid delivery apparatus and in particular to apparatus for the infusion of medical liquids into patients.

Infusion pumps are known for use in delivering medical liquids contained initially in a syringe into the body of a patient at a pre-determined rate. Many infusion pumps are designed to take only one size of syringe and those pumps that will take more than one size are usually limited to one particular make of syringe.

UK Patent 1178738 discloses an arrangement for clamping a syringe in an infusion pump which includes an upstanding U-shaped bracket having a lower leg attached to a base part. The two legs of the U-shaped bracket carry a bolt which has an internally threaded clamp arm. The vertical base of the U-shaped bracket is sufficiently long to ensure that the clamp arm can be screwed upwardly a sufficient distance to clear the largest syringe to be accommodated in the infusion pump.

A particular disadvantage of this known clamping arrangement is that the syringe can only be loaded conveniently into the infusion pump when said infusion pump is horizontal. Further, the loading process is cumbersome and requires the use of both hands.

UP Patent 1026593 discloses a syringe clamped to an infusion pump by means of a wire arm having a coiled portion slidable along a vertical supporting staff. When the wire arm is tilted, the friction between the coiled portion and the staff is sufficient to hold the arm fixed and so clamp the syringe.

Although this clamping arrangement can accommodate different diameters of syringe the loading process is cumbersome and requires the use of both hands. Furthermore, it would be difficult to use if the infusion pump were placed in the vertical position.

A similar clamping arrangement is described in UK Patent 1465797 in which an L-shaped rod holds a syringe barrel against a mounting piece on an infusion pump.

It is an aim of the present invention to provide an infusion pump which has a clamping arrangement for receiving syringes of different sizes; and which can be loaded using only one hand regardless of the orientation of the infusion pump.

It is a further aim of the present invention to provide an infusion pump having a clamping arrangement incorporating means for sensing the diameter of the syringe being fitted.

According to the present invention an infusion pump comprises a body casing and a clamp mounted thereon for movement between a closed clamping position and an open position thereby permitting different sizes of syringes to be held between the body casing and the clamp. The infusion pump is characterised in that the clamp is pivotally mounted on the body case and that means is provided for biasing the clamp towards its closed position.

In a preferred embodiment, the clamp includes a shaft which is pivotally mounted on the body casing, a potentiometer being rigidly connected to the shaft for sensing the pivotal movement of the shaft and hence the size of a syringe mounted in the infusion pump.

An embodiment of the invention will now be described, by way of example, reference being made to the Figures of the accompanying diagrammatic drawings in which:-

Figure 1 is a perspective view showing a syringe held in place by a clamp in an infusion pump;

Figure 2 is a partial front elevation of Figure 1;

Figure 3 is a partial front elevation as shown in Figure 2 but with the syringe removed;

Figures 4a to 4d are end views illustrating a method of loading a syringe into the infusion pump of Figure 1;

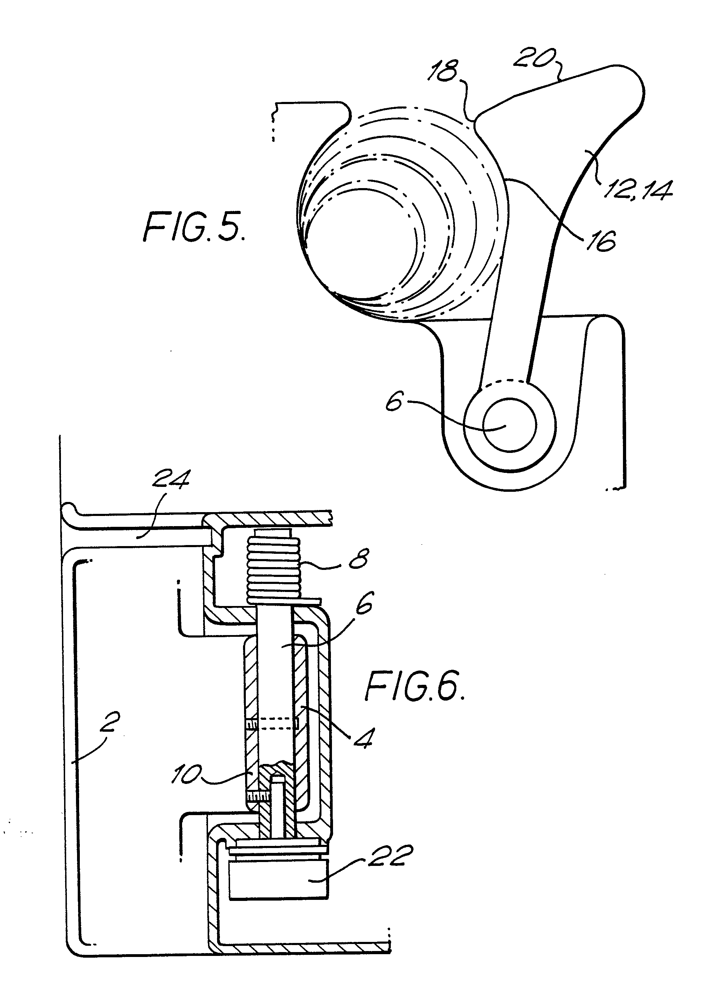

Figure 5 is a sketch illustrating an arm of the clamp shown in Figure 1 and its relative positions when accommodating syringes of different sizes; and

Figure 6 is a partial cross-section through sensing means forming part of the clamp shown in Figure 1.

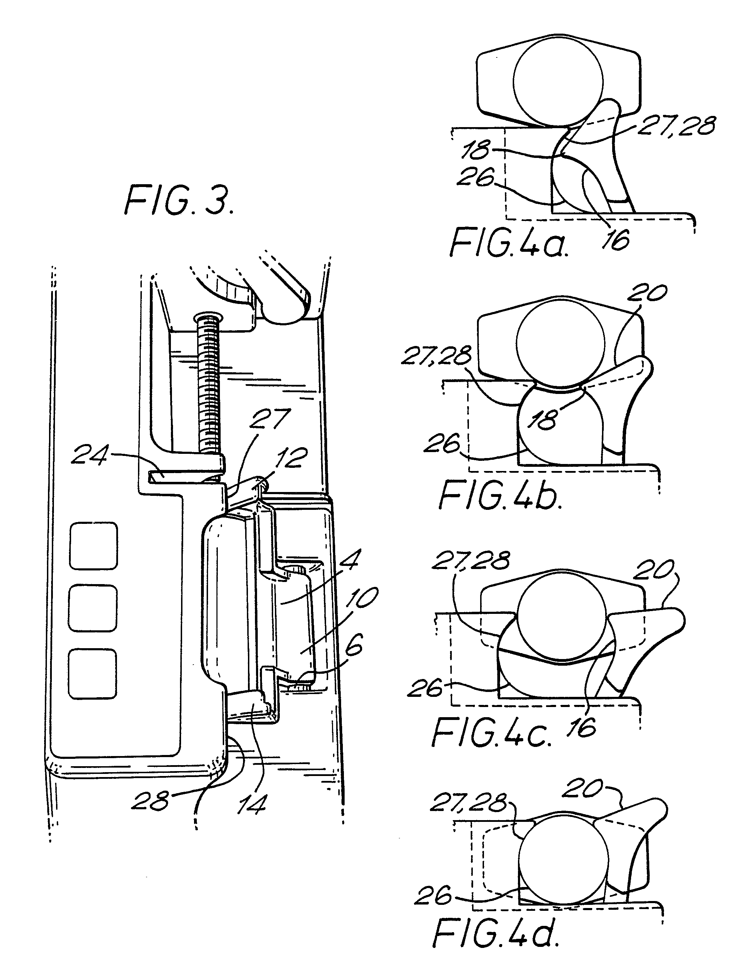

As shown, an infusion pump 1 has a body casing 2 to which is pivotally mounted a clamp 4. The clamp 4 includes a shaft 6 which, as best seen in Figure 6, is pivotally mounted on the body case 2 and is biased by a spring 8 to a closed position best shown in Figure 4a. The clamp 4 consists of a central part 10 and at each end of the central part 10 two substantially identical arms 12, 14. Each arm 12, 14 has a configuration best illustrated in Figures 4a to 4d which includes a radiused surface 16 for engaging the surface of a syringe 100, a heel 18 and a flat surface 20.

Rigidly attached to one end of the shaft 6 is a potentiometer 22.

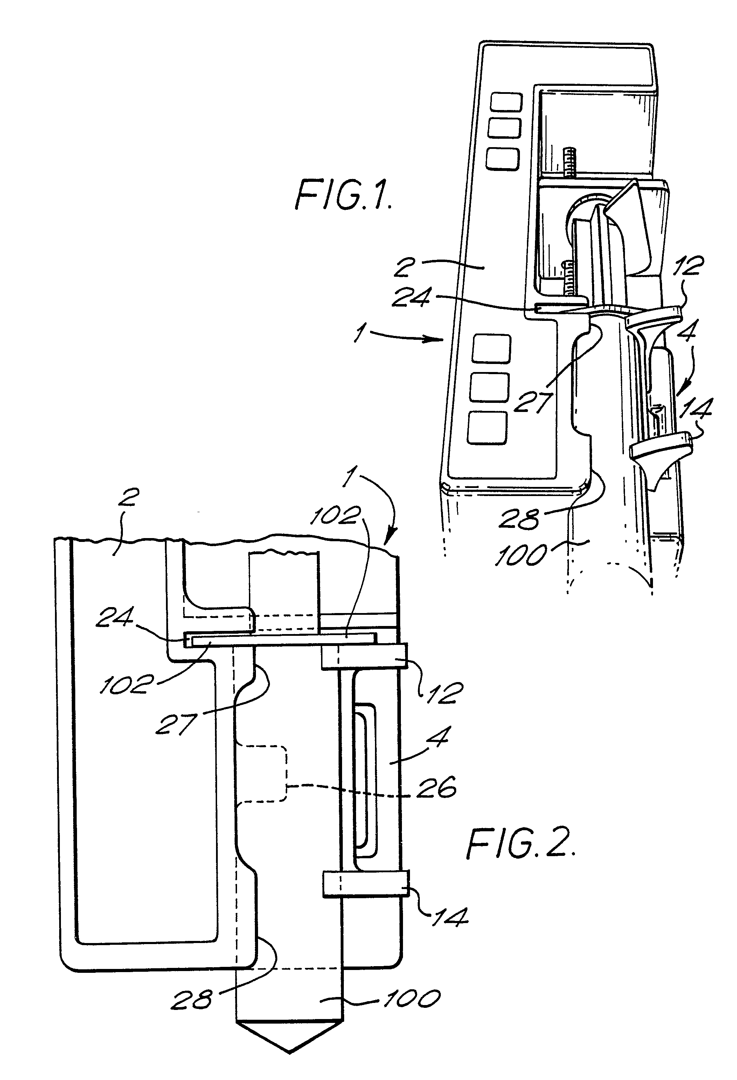

The body case 2 is provided with a slot 24 for accommodating a flange 102 on the syringe 100 when loaded in the infusion pump 1. The body case 2 also includes a radiused support lug 26 which as best illustrated in Figure 2 is located at a position spaced from but substantially mid-way between the arms 12, 14 to give stable three point location of the syringe body 100.

In use, when it is desired to load the syringe 100 into the infusion pump 1, the syringe 100 is first placed in the space between the body case 2 and the clamp 4 with the flange 102 located in the slot 24 and against the upper (as shown in Figure 2) surface of the upper arm 12.

Next, the syringe 100 is pushed so that it engages the surface 20 of each arm 12, 14 thereby pivoting said arms clockwise as shown in Figures 4a to 4d. The angle of the surface 20 is arranged to give the necessary mechanical advantage required to overcome the torsion of the spring 8.

As shown in Figures 4a to 4d, the syringe 100 eventually clicks into place between the surfaces 16 of the arms 12, 14 and the radiused surface of the lug 26 on the body case 2.

It will be evident, that the heels 18 on each arm 12, 14 prevent unintentional removal of the syringe 100 from the infusion pump 1. Further, one surface of the syringe flange 102 is now fixed in the slot 24 whilst the other surface rests against the upper surface of arm 12 to resist the pushing force of the syringe plunger (not shown).

In the clamped position illustrated in Figure 4d the arms 12, 14 together with overhanging lugs 27 and 28 on body case 2 will enclose 290° or more of the syringe diameter depending on the size of the syringe 100.

It will be evident, that different diameters of syringes will cause the clamp 4 to find different angular positions relative to its pivot point, that is the longitudinal axis of shaft 6. By rigidly fixing the potentiometer 22 onto the end of the shaft 6, the potentiometer will accurately sense the position of the clamp thereby monitoring the size of the syringe mounted in the infusion pump.

The potentiometer can also be arranged to detect excessive movement during infusion before the syringe becomes fully dislodged from the pump and the potentiometer can be connected into an audible or visual alarm system.

An advantage of the embodiment described above is that a syringe can be loaded into the infusion pump 1 single handed when the infusion pump is either vertical or horizontal.

## Claims

1. An infusion pump comprising a body case 2 and a clamp 4 mounted thereon for movement between a closed clamping position and an open position thereby permitting different sizes of syringe to be held between the body case 2 and the clamp 4, characterised in that the clamp 4 is pivotally mounted on the body case 2 and means 8 is provided for biasing the clamp 4 towards its closed position.

2. An infusion pump as claimed in claim 1, charactersied in that the clamp 4 includes two spaced, substantially identical arms 12, 14 each having a radiused surface 16 for engaging the surface of a syringe 100.

3. An infusion pump as claimed in claim 2, characterised in that the arms 12, 14 each have a flat surface 20 which is engaged by the syringe 100 when loading the syringe 100 into the infusion pump 1, said flat surface 20 offering a mechanical advantage in order to overcome the biasing means 8 and thereby permit the clamp 4 to move towards its open position.

4. An infusion pump as claimed in claim 2 or 3, characterised in that each arm 12, 14 has a heel 18 for retaining a syringe 100 in the infusion pump 1.

5. An infusion pump as claimed in any one of claims 1 to 4, characterised in that the clamp 4 includes a shaft 6 which is pivotally mounted on the body case 2, a potentiometer 22 being rigidly connected to the shaft 6 for sensing angular movement of the shaft 6 and hence the size of a syringe 100 mounted in the infusion pump 1.

FIG.1.

FIG.2.

EP 0 388 102 A2

FIG. 3.

FIG.4a.

FIG.4b.

FIG.4c.

FIG.4d.

FIG.5.

18    20

12,14

16

6

FIG.6.

24

8

6

2

4

10

22